# EUROPEAN PATENT APPLICATION

(11) **EP 3 081 635 A1**
(43) Date of publication of application: **19.10.2016**
(21) Application number: 16169346.0
(22) Date of filing: 29.10.2013
(51) Int. Cl.: C12N 1/12, C12M 1/00, C12M 1/04, C10L 1/02, C11B 1/00, C10G 1/02, A23K 10/12

(54) **PROCESS FOR PRODUCING BIOMASS AND PRODUCTS DERIVED THEREFROM BY CULTIVATING UNICELLULAR ALGAE IN AN AQUEOUS MEDIUM SUPPLIED WITH A CO2 CURRENT, AND PLANT DESIGNED FOR THIS PURPOSE**

(30) Priority: 30.10.2012 ES 201231661
(62) Divisional of application: 13850180.4
(71) Applicant: Biosinkco2 Tech Lda, 9000-019 Funchal, Madeira (PT)
(72) Inventor: ESCUDERO CAMPILLO, Pedro, 9000-019 Funchal, Madeira (PT)
(74) Representative: Pons

(57) **Abstract**

The invention relates to a process comprising cultivating unicellular algae in an aqueous medium in a set of photobioreactors with injection of a gas current containing CO₂, characterised in that it also comprises accumulating the culture of unicellular algae following the extraction thereof, and subjecting said culture to successive treatment steps by which means biomass with a humidity degree of between 0 % and 90 % is obtained (mechanical and/or chemical extraction), and subsequently said biomass is subjected to extractions of lipids and/or fatty acids and a first reject (decantation and/or cavitation), of biooil and/or bio-crude and a second reject (low-temperature direct heat liquefaction) and of biofuel (cavitation, decantation and/or heat treatment), in such a way that the various rejects are stored, treated physicochemically and reincorporated into the process as a raw material. The invention also refers to a facility of photobioreactors designed for such a purpose, comprising separate chambers for each biomass and product extraction method, and for storing the rejects.

## Description

### FIELD OF THE INVENTION

The present invention falls within the field of modern biotechnology applied to algae, specifically Phycotechnology. More specifically, it falls within the area of developing clean technologies for purifying and capturing harmful gases or greenhouse gases, as well as in the area concerned with producing biomass from unicellular algae for the industrial manufacture of processed and energetic products for consumption. The main application focuses on the transformation of greenhouse gases, especially CO₂, into biomass by means of the (ultraintensive) cultivation of certain species of unicellular algae, including several different strains simultaneously in monoculture, in closed systems such as vertical annular photobioreactors. This makes it possible to obtain from the biomass other products that are incorporated into the food chain, mainly lipids and proteins, before obtaining final biofuel.

In this way the intentions is to achieve an energy-efficient process and prevent the emission of liquid and gas pollutants into the environment.

### STATE OF THE ART

It has been found that average energy needs at the global level have now increased from 2300 kcal/day per person in 1980 to 2800 kcal/day per person in 2010. Furthermore, this figure is estimated to reach 3100 kcal/day per person by 2030. This, combined with the population growth that is being recorded, has pushed us to search for alternatives that can complement traditional food chain systems.

In parallel, there is growing concern about the thinning of the ozone layer and to the need of reducing emissions of greenhouse gases, among which the most important are CO₂ and CH₄. Finally, the post-petroleum era is coming closer and closer, and for this reason we are working on finding energy alternatives that are effective and not largely dependent on weather conditions.

Unicellular algae do not compete with any current staple food, they have an outstanding reproductive capacity and only need sun, a few nutrients and CO₂, in addition to water, in order to be cultivated. This water should be salty, so that they do not compete with the drinking water needs of the population.

Patent application EP 2371940 A1 discloses a process and an installation for the production of biofuels and reduction of CO₂ from punctual sources of emission. To this end, it employs non-annular tubes measuring less than 0.1 m in diameter and up to 80 metres in length. This does not lead to equal irradiation conditions across the tubes, resulting in a low surface/volume ratio, which in turn causes biomass production to decrease proportionally. In addition, the treatment of the biomass obtained in the process is carried out conventionally, wherein compounds and processed products such bio-oil or biofuel are obtained jointly.

This kind of process, such as that presented in application EP 2371940 A1, requires a drying step between the steps of separation of the biomass and the extraction of compounds, which is energy inefficient.

Document US 2008086938 A1 focuses on the overall description of the CO₂ collecting process and subsequent recycling into biofuel. In this case, also after obtaining biomass, biofuel is obtained in a single process.

International patent application WO 2007147028 A2 describes the necessary equipment for capturing CO₂ and introducing it into photobioreactors, mentioning a separation process, and describing a final extraction of biomaterials, although the claims only explain constituent elements of the CO₂ collecting system and their introduction into the photobioreactors, as well as the different photobioreactor options.

Document ES 2370583 A1 discloses photobioreactors and accumulation tanks, without mentioning the rest of the process to obtain biomaterials.

Patent application US 4868123 A relates only to the photobioreactors, but does not introduce the improvements discussed in this patent application that make it much more efficient. This document only relates to a type of photobioreactor for cultivating microalgae which operates differently than that which is disclosed in the present invention, not including a cleaning system or the possibility of all air-water operation, in parallel and crossover. Additionally, it does not mention any sort of subsequent process to that of cultivating microorganisms.

Document ES 2356653 A1 describes an invention with conical photobioreactors that are submerged inside a tank.

Document US 2007048859 A1 includes the steps of cultivating microalgae from the cultivation area with photobioreactors, CO₂ collecting and subsequent treatment of the biomass to obtain a final product based on the biofuel. To this end, it uses photobioreactors without a cleaning system and in a single arrangement, conventional drying systems and a single thermochemical treatment for obtaining biofuel without additional processes for obtaining value-added products. All of this is intended to be carried out without any combination of species or the reuse of rejects.

In light of the aforementioned documents, the present invention aims to improve the efficiency of the processes for obtaining biomass from unicellular algae and extracting value-added products therefrom, by improving on the sequence of the steps of obtainment, separation and extraction, thus making it possible to carry out the process while wet, or to recover the rejects of the process in order to treat them and subsequently reintroduce them into the water and gas lines, as well as by improving the facilities designed for this purpose.

### GENERAL DESCRIPTION OF THE INVENTION

The present invention relates to the process for the full conversion of harmful gases, such as greenhouse gases, into products from which subsequent industrial benefit may be derived in the energy and food sectors, both for humans and for animals, by the ultra-intensive cultivation of unicellular algae and with the addition of sea, fresh and/or salty water, sunlight and nutrients, all by means of a closed cycle and making use of all the surplus in each of the stages.

Specifically, the invention is directed at a process for producing biomass and products derived therefrom by cultivating unicellular algae in an aqueous medium supplied with a CO₂ current in an assembly of photobioreactors, comprising:
- injecting a current of sea, salty or fresh water into the photobioreactors as a culture medium for the algae, previously enriched with nutrients, microfiltered and disinfected,
- injecting a strain of at least one species of unicellular algae into the photobioreactors, and placing said strain in contact with the enriched water current under light conditions for the culture to grow by means of photosynthesis;
- injecting a current of gases containing CO2into the photobioreactors, characterised in that it further comprises
- extracting part of the algae culture from the photobioreactors, storing in a accumulation tank and sending to a separate unit to extract the biomass by means of mechanical and/or chemical separation, such that the obtained biomass has a humidity degree comprised between 50 % and 90 %;
- subjecting the wet biomass to decantation and/or cavitation in a separate unit in order to extract part of the lipids and/or fatty acids and other added-value products along with a first reject, passing the remaining biomass on to the following step;
- subjecting the remaining biomass from the preceding step to direct, low-temperature, heat liquefaction in a separate unit in order to produce a biocrude and a second reject, all this at a temperature comprised between 250 °C and 350 °C and a pressure comprised between 150 and 210 bar, both limits included, for a time comprised between 1 and 120 minutes;
- recovering the rejects from both the step of lipid, fatty acid and other added-value product extraction and the subsequent step of thermochemical treatment under subcritical conditions, storing each reject in separate chambers and subjecting to physico-chemical treatment based on adjusting salinity, pH, microfiltration, disinfection and addition of nutrients, for subsequent reintroduction into the culture.

Biocrude should be understood as the renewable equivalent of a heavy petroleum-fossil fuel oil, in the present case, however, obtained from algae biomass. Refining biocrude makes it possible to obtain a large variety of compounds, such as naphtha, biofuel and bio-oils, etc.

The present process holds many advantages over the state of the art, mainly the accumulation and subsequent treatment of the algae culture extracted from the photobioreactors in wet conditions (wet biomass), as well as recovering all the rejects or surplus generated in the various mechanical and/or chemical separation processes used to obtain lipids and/or fatty acids and/or added-value products, the subsequent heat treatment and final optimisation process for the end biocrude.

Another improvement resides in the process for treating the biomass once it has been extracted, in order to obtain added-value products such as lipids and fatty acids and other added-value products, such as vitamins, antioxidants such as beta-carotene and astaxanthin, hormones, etc., which may be used in the agro-food, pharmaceutical and cosmetics or biomedical industries, following to guidelines set out in " Selective extraction of carotenoids from the microalga Dunaliella salina with retention of viability" (Hejazi MA, de Lamarliere C, Rocha JM, Vermuë M, Tramper J, Wijffels RH; Biotechnol Bioeng. 2002-Jul) or in " Production of cell mass and eicosapentaenoic acid (EPA) in ultra high cell density cultures of Nannochloropsis Sp. (Eustigmatophyceae)" (NingZoy, Chengwu Zhang, Zvi Cohen & Amos Richmond, 2000; European Journal of Phycology, 35:2, 127-133). Lipids, fatty acids and other added-value products are produced before the biocrude is extracted rather than in the same single-step method, as is the case in conventional processes (for example EP 2371940 A1). The method for extracting lipids, fatty acids and other added-value products, using either decantation and/or cavitation means, is carried out prior to the heat liquefaction process, wherein biocrude is extracted. In the present invention, the three processes cited are clearly separated and differentiated from one another and are performed in independent units and machines, which largely optimises the results in comparison with those obtained when the processes are carried out jointly, in addition to enabling the complete recovery of the rejects from the various processes, see "Hydrothermal Treatment (HTT) of Microalgae: Evaluation of the Process as Conversion Method in an Algae Biorefinery Concept" (Laura García Alba, Cristian Torri, Chiara Samori, Jaapjan van der Sped, Daniele Fabbri, Sascha R.A. Kersten and Derk W.F. Brilman (2011). Thermo-Chemical Conversion of Biomass Group, Faculty of Science and Technology, University of Twente, P.O. Box 217, Enschede, The Netherlands).

Furthermore, the fact that this process is carried out in consecutive steps makes it possible to start with a wet extracted biomass, without the need for drying, with a high degree of humidity, in fact, to obtain products such as lipids, fatty acids, added-value products, biocrude and, if possible in preferred cases, finally biofuel.

Recovering waste generated in these processes, for example the first reject produced in the mechanical and/or chemical biomass extraction step, from the steps in which lipids, fatty acids and other added-value elements are extracted, as well as from the reject produced in the steps in which biocrude is obtained, makes a relevant contribution to the development of this kind of methods, since the reintroduction thereof into the process as a raw material, following a prior conditioning process, increases the efficiency of the overall process considerably. The waste obtained in these steps fundamentally, and in by a large percentage, are a culture with a lower concentration of algae after the initial mechanical and/or chemical separation. Their conditioning by physico-chemical treatment thereof is based on the adjustment of salinity, pH, microfiltration, disinfection, addition of nutrients and agitation, for subsequent reintroduction into the culture. Between 50 % and 99 % of culture which is periodically extracted and treated for biomass extraction becomes a reject product for next step and thus is returned to the culture after the prior treatment discussed in the present paragraph.

The present process does not uses antibiotics or fungicides, so that the obtained products have a higher quality that the conventional processes in which they are used (see, for example, international application PTC/ES2007/000733).

Another advantage worth noting is the use of different species in the same biomass production and treatment plant, as a monoculture (each one separated in a different area), such as a lipid-rich species and a second low-lipid species, which complement each other to produce a fuel that is rich in hydrocarbons.

A second object of the present invention consists of a plant for producing biomass and products derived therefrom by cultivating at least one unicellular algae species in aqueous medium supplied with a gas current containing CO₂ in a set of photobioreactors according to the process described in any one of the preceding claims, comprising:
- at least one unit for the collecting, storage and injection of the sea, fresh and/or salty water current connected to a nutrient injection unit that injects nutrients into said current before entering the photobioreactors, and to a microfiltration and disinfection unit;
- at least one unit for the collecting, storage and injection of the gas current containing CO2 into the photobioreactors;
both units being connected to
- a set of two or more photobioreactors of annular vertical and circular type connected to each other for producing biomass by means of cultivating at least one species of unicellular algae;
characterised in that it further comprises
- means for extracting part of the culture from inside the photobioreactors and an accumulation tank thereof, connected to a mechanical extraction unit and/or to a wet biomass chemical extraction unit;
- a unit for extracting lipids and/or fatty acids and/or other added-value products by means of cavitation, decantation and/or dissolution of the biomass; connected to
- a thermochemical treatment unit for producing biocrude by means of direct, low-temperature, heat liquefaction from the surplus biomass in the unit for extracting lipids and/or fatty acids and/or other added-value products;
- a storage chamber for the first reject produced in the mechanical extraction unit and/or in the chemical extraction chamber, with means for recirculating said reject to the unit for injecting the water current into the photobioreactors after conditioning thereof inside the storage chamber; and
- a storage chamber for the second reject produced in the unit for extracting lipids and/or fatty acids and/or added-value products and in the thermochemical treatment unit, with means for recirculating said reject to the unit for injecting the water current into the photobioreactors after conditioning thereof inside the storage chamber.

The described plant makes it possible to carry out the process object of the present specification optimally, reaching values that are 7 times greater than the surface/volume ratio of the plant, thus increasing the production of biomass by this amount, which amount is higher than those recorded in the state of the art. It has been found that the optimum ratio of the plant described is up to 180 litres/m² of culture in the seeding area as opposed to the 25 litres/m² of culture known in the state of the art, with conventional photobioreactors that are similar to that of the present specification.

### DETAILED DESCRIPTION OF THE INVENTION

Regarding the biomass production process, it is worth noting that the gas current may be a current made up entirely of CO₂, or as an alternative may be a mixture of gases from a combustion process.

Optionally, the gas current which is a mixture may be washed, compressed, and the CO₂ may be separated from the rest of the substances contained therein, such as SOx, NOx, and ashes, prior to being introduced into the photobioreactors. This adaptation of the gas current prior to being injected is performed by cooling the gases if needed so that the maximum temperature thereof is always bellow 40°C, and separation thereof is performed according to the aforementioned types. Also preferably, but not by way of limitation, gas current may be mixed with compressed air prior to being introduced into the photobioreactors, regardless of whether said current is only CO₂ or a mixture of gases.

In a particular embodiment, the gas current is injected radially into the photobioreactor. Preferably, said injection is performed by means of a diffuser that is integrated between the tubes making up the photobioreactor, in a radial fashion, and covering the entire perimeter of the tube, said diffuser being placed in the mean radius between outer tube and the inner tube with holes drilled in it so as to enable the CO₂ to exit with or without compressed air. Said injection is preferably carried out discontinuously, in pulses with a duration comprised between 1 and 3600 seconds at time intervals comprised between 1 to 3600 seconds, both limits included. This discontinuity, together with the cycles of the cells in the culture, promotes fixing of greenhouse effect gases.

Since both chemical and biological collecting by unicellular algae of the gas current containing CO₂ is between 45 % and 60 %, a system for collecting surpluses that come out the top of the photobioreactors may be incorporated in the plant, for subsequent reintroduction thereof into the plant's gas line via the corresponding plant compressors, mixing tanks and piping and associated control system. The gas collecting efficiency increased up to a value comprised between 60 % and 90 % with this alternative.

Preferably, the gas current is injected by an amount comprised between 0.2 m³/m³ of culture and 2 m³/m³ of algae culture.

After water collection, it is subsequently (micro-) filtered depending on its salt content, especially if the water is seawater, along with removal of organic material and oily products, and micro-filtering of clays and other undesired elements in accordance with the type of unicellular algae being cultivated.

The water current may be enriched with macronutrients, micronutrients and/or trace elements, in a percentage comprised between 10 % and 30 % by weight of the obtained biomass. Specifically, nutrients may be selected from nitrogenised salts such as nitrate and ammonium, phosphates and any combination thereof.

In a preferred embodiment and alternatively to the usual air-lift in the operations of this kind of photobioreactors in terms of the liquid-air flow conditions, the water current can be rotated inside the photobioreactors in the opposite direction to the injection of the bubbles with the gas in question when both are put in contact. This enhances the absorption of the gas by the unicellular algae. It is a chemical effect consisting of the fact that, by opposing the flow of the fluid to that of the CO₂ bubbles, a larger collecting surface is obtained, and absorption yield is improved.

Commonly, but not by way of limitation, the strain is introduced into the plant through one of the photobioreactors, diluting it in the sea, fresh and/or salty water through the existing pump system. It subsequently spreads out from this photobioreactor, 50 % being extracted from the remainder when the concentration is suitable, and seawater is always added to fill out the volume of the photobioreactor.

Preferably, culture is maintained inside the photobioreactors at a temperature comprised between 5 °C and 45 °C, still more preferably between 15 °C and 35 °C.

In the most preferred embodiment, the algal culture is kept inside the photobioreactors until reaching a concentration comprised between 100 million cells/ml and 600 million cells/ml. Thus an amount of daily culture, which is comprised between 2 % and 50 % of the total volume inside the photobioreactors, may be extracted from the photobioreactor. In a more preferred embodiment, said portion of the algal culture is accumulated or stored after extraction with constant stirring and aeration or air and CO₂ supply, which makes it possible to achieve different reactions depending on the characteristics of collected culture.

Mechanical separation of the biomass may be performed by means of one of the processes selected from the group consisting of centrifugation, super-centrifugation, decantation and/or filtration, and chemical separation is performed by means of flocculation. If both mechanical and chemical actions are performed in the process, then each of them is carried out in separate units, such that preferably chemical separation of the biomass is performed first and mechanical separation performed second in a sequential way, thus reducing the level of culture in the mechanical separation process with consequent improvements in process efficiency.

It is worth pointing out that the biomass that is left over from the mechanical and/or chemical separation processes and that of the extraction of lipids and other products, which is used to obtain bio-crude may be comprised between 80 % and 99 % of the total biomass, since in the previous stage only between 1 % and 20 % of total treated biomass has been extracted.

Also in the most preferred embodiment, the first reject, from the biomass separation step, and the second reject, from subsequent processes to obtain lipids and/or fatty acids and/or value-added products and bio-crude are stored under continuous stirring by means of propeller stirrers that ensure complete homogenisation of the product in less than 2 hours, and which may be vertical and/or horizontal, with one or more blades depending on the tank and with aeration or a mixture of air and CO₂, and is conditioned before being reincorporated into the line of water to inject into the photobioreactors.

As a result of reintroducing of rejects of the conditioned form back into the process and as a product generated in certain moments and under certain temperature and aeration conditions by itself, a film of foam is formed on the top layer of the water current inside the photobioreactors. Thus, in an optional embodiment, the foam may be collected, said foam containing biomass and/or nutrients in a percentage comprised between 1 % and 5 % of the total existing volume in the photobioreactors.

In a preferred embodiment of the process, the strain of unicellular algae is selected from the group consisting of eukaryotic microalgae and in particular Cyanobacteria, Chlorophyceae, Rhodophyceae, Eustigmatophyceae, diatoms, Dinophyceae, Bacillariophyceae, Xanthophytes and Phaeophyceae, and any combination thereof.

In the most preferred embodiment, bio-crude is subjected to cavitation, decantation, pressing and/or heat treatment in a separate unit to obtain biofuel (distilled and purified bio-crude). The reject of this step can also be stored, treated and reintroduced into the process like the other rejects and in the same conditions described for them.

With regard to the biomass production plant described in the previous section, it is worth noting that the gas capturing unit can be nourished by a current of greenhouse gases, for example coming directly from the smoke stacks of the industrial facilities that emit them. This unit is preferably formed by take-offs from smoke stacks, duct networks, automatic operating systems, accessories and extractors.

The plant may further comprise a compressed air injection unit connected to the unit for collecting, storage and cleaning, and injection of the current of gases containing CO₂, prior to entering the photobioreactors. The plant may further comprise an accumulation unit for the gases to be injected, so that if the main supply source thereof fails, the process may continue. The capacity of these tanks is such that they allow normal operation of the plant without the usual supplies from one month to four months.

The water current collecting, storage and injection unit is made of the consequent systems of pipes, electric pumps and filters, as well as control elements. Preferably, the accumulation tank of sea, fresh and/or salty water has a capacity of between two sixths and four sixths of the total capacity of the culture volume of the plant. The control elements are pH and temperature probes, a conductivity probe, turbidimeters and maximum and minimum levels. This, together with the electrically operated valves associated to the plant management system, enables the sea, fresh and/or salty water that is to be introduced into the feeder tanks of the photobioreactors enter in the ideal conditions.

Optionally, the plant comprises from 2 to 5000 photobioreactors. Said photobioreactors are preferably constituted by an outer tube and an inner tube delimiting a space between them, wherein the water and gas currents are injected and where the unicellular algae are cultivated, said space being divided into longitudinal odd number sections from 1 to 9, and the span of the passage inside the tubes being smaller than 5 cm The tubes may have a height equal to or greater than 10 meters. Because the tubes are divided into 3, 5, 7 or 9 longitudinal sections or compartments, the functioning of the light-dark binary cycles is exactly the same throughout the whole section of the tube, therefore achieving much greater efficiency and more control than that which is achieved with the different areas present in a normal cylindrical tube. The inner and outer tubes may preferably be made of plastic materials, both rigid and flexible, with the particular feature that the inner tube may or may not be translucent and that the inner cavity may or not may be used to adjust the temperature of the existing culture in the ring.

Even more preferably, the photobioreactors further comprise a translucent concentric tube covering the outer wall of the outer tube. This tube made of transparent material prevents the sun from shining directly onto the outer tube containing the culture and thereby may prevent the photoinhibition effects that occur with high levels of irradiation and greatly reduce the energy requirements for adjusting the temperature of the culture, as well as number of other associated improvements such as reduction in thickness, in turn lowering the production cost of the tubes.

In a particular embodiment of the invention, the set of the photobioreactors comprises:
- an area for mixing the gas current, the water current and a strain of the algae to be cultivated;
- an area for growing the algae culture; and
- an area for extracting the culture from inside the photobioreactors.

Each area is comprised of specialised photobioreactors for each function, the three areas are connected together in the logical order: the mixing area is connected to the culture growing area and, in turn to the extraction area. That is, the plant has photobioreactors which are used for the mixing or feeding, others which are used for growing, and other final ones for the culture collection. This is achieved with the different arrangements of the tubes inside each photobioreactor, thus varying the separation between them, water-sheet thickness, culture conditions and a number of other parameters allowing for optimal sequential development to optimise the process, as has been explained above.

The photobioreactors may be connected to each other by means of pipes buried under ground where the plant is located, the water current with the culture being driven from one photobioreactor to another by the electric pump.

The photobioreactors may further comprise electric pumps which are associated to all the network of pipes that interconnect the cultivation area, containing a pump for each group of photobioreactors. With the electrically operated valves installed in the pump zone, it is possible to use the controls to run the system explained in the preceding paragraph and/or in the present one and all others that may be possible. Each one of these pumps makes the water spin in the opposite direction to that of the gas current injection when both are put in contact. In another alternative operating option, the same pump is the one which recirculates the culture through the buried pipes, thus favouring the heat exchange with the ground (at a constant temperature) and thus achieving the highest productivity of the process with the lowest possible energy consumption. In this case, culture recirculation varies between 10 % and 60 % of the unitary volume.

The arrangement of the tubes in the set of the photobioreactors shall be that which enables maximal development of the unicellular algae depending on their degree of concentration with respect to the irradiation and temperature at that time. Since these parameters oscillate both through the year and depending on the location of the plant, different alternative designs are combined, while other parameters also vary, such as residence time in each of the configurations, daily extraction percentage, the percentage of recirculation flow over the total volume and nutrient and CO2supply, in order to obtain the best values for reproduction. Specifically, the arrangement of the tubes or columns in the photobioreactors and in the cultivation area is adapted to the different needs of the culture depending on the time of year and environmental conditions. There are, in the state of the art, photobioreactors formed by tubes with a linear arrangement, others with circular arrangements, and others with double arrangements of these both, such that the distances between the components also vary, thus combining the different possibilities of irradiation/passage span/speed of CO2 and air mixture, to always obtain the best production. In the case of the present invention, as a consequence of the division into areas of the algal culture, the plant is stratified according to the environmental conditions and the different reproductive phases of the culture, so that the plant may be adapted to each time of the year, achieving the best possible efficiency. Irradiation needs in one time of the year are very different from those required in other time, for which reason the path of the culture is adapted from its initial seeding to extraction, and between one extraction and the next. That which has been indicated corresponds to the principles demonstrated in the technical article " Design principles of photo-bioreactors for cultivation of microalgae", by D. Clemens Posten, Institute of Life Science Engineering, Division of Bioprocess Engineering, University of Karlsruhe, Strasse am Forum 8, D-76131 Karlsruhe (Germany), DO1:10.1002/elsc.200900003, 29 May 2009 , and in " Microalgal Photobioreactors: Scale-up and optimization", Barbosa, M.J.G.V., 2003.

Optionally and preferably, the photobioreactors further comprise an automated device for cleaning the walls of the tubes delimiting the space between the inner tube and outer tube, said cleaning device being constituted by an elastomeric scraper whose plan corresponds to the profile of the space between the tubes and a thickness comprised between 2 and 5 cm, with a central through hole that has a lower frustoconical section and an upper cylindrical section, the hole having the overall shape of an inverted funnel; and a sphere made of plastic material with a larger diameter than the diameter of the cylindrical section of the hole, which moves inside the space defined by the lower frustoconical section of the through hole. The incorporation of this cleaning system allows the plant to operate without the need to empty the photobioreactors, which simplifies maintenance of the facility and efficiency thereof with respect to the prior art. The cleaning device operates as follows:
1.- In the normal position it is at rest at the bottom of the tube, in the cultivation area, i.e., between the outer and the inner tubes.
2.- Once it becomes desirable to activate it, the flow direction of the culture is changed from its normal operation (the culture enters through the top part and is collected at the bottom) to the opposite direction, and should the operation being used that in which it is fed from bellow and spills over the top, the flow rate is increased so that the device is activated once it is enough to make the inner sphere rise.
3.- The operating time of the device, which is activated because of the changes to the electrically operated valves found in the area of each of the culture pumps, is such that it makes it possible to make the cleaner go upwards without butting up against in the top cover of the photobioreactor.
4.- Once the cleaner reaches the top, this option is inhibited, such that it returns to the previous state, and falls under its own weight (its density is greater than that of the culture water) back to its original position.

The operation may be repeated from 1 to 50 times a day depending on the internal cleaning state of the tubes, which in turn depends on the species being employed and the conditions under which it is being cultivated.

Ultimately, annular vertical tubes are used as photobioreactors, it being possible for a third outer layer to be arranged in different ways inside the photobioreactors, which enable the cultivation area to adapt to existing climatic conditions in each period, differentiating culture recovery areas, mixing areas and the various growth areas prior to the extraction area. This equally results in maximum CO₂ absorption being attained in each one of the current environmental conditions. These aspects, in addition to improving both the collection and reproduction efficiencies of the unicellular algae, improve the overall performance of the system, preventing fooling (prokaryotes and unicellular algae stuck to the outer tube) from forming and largely reducing maintenance operations owing to the incorporation of a cleaning system for the tubes, without it being necessary to empty the same.

As described above, a foam film may be form on the upper layer of the water current inside the photobioreactors, especially as result of the reintroduction of the reject from the various biomass treatment stages, until the end products desired are obtained. This foam film may even be generated naturally inside the photobioreactors, in certain conditions and at certain times during the reproduction process, even prior to introducing the treated rejects, which contain a high percentage of nutrients and/or biomass. For this reason, the photobioreactors may further comprise means for collecting the foam, based on an upper channelling of the appropriate dimensions according to the volume of the photobioreactor, conveniently driven to an accumulation area generated for this purpose in the plant and drive means (pipes and electric pumps), which drive said foam to the foam accumulation tank for foam coming from the culture extracted from the photobioreactor. It will subsequently be reintroduced back into the photobioreactors supply tank or to the clarification tank, according to the treatment applied and depending on the unicellular alga being cultivated.

The top portion of the photobioreactors may also comprise means for collecting the excess CO₂ not consumed by the algae culture and means for driving said excess gas from the gas collecting and injecting unit to the photobioreactors, in order for them to be reintroduced.

The capacity of the unit for accumulating the culture extracted from the photobioreactors is preferably comprised between half and one tenth of that of the total volume of the plant. It may also have stirrers and supply means with CO₂ and/or compressed air and measurement and control systems, to be integrated into the plant operation. The control elements include pH and temperature probes, conductivity probe, turbidimeters, maximum and minimum levels and nutrient control probes.

In order to attain optimal biomass production, the storage units for the first and second reject may comprise stirring and aeration means or means for injecting air and CO₂ inside, in order to improve the behaviour of the culture and/or the subsequent reincorporation into the cultivation area thereof, it being possible to produce decantation and/or variations in the composition as required. The capacity of each of the reject storage units is normally between half and one tenth the capacity of the total volume of the plant. Optionally and preferably, when both rejects have been treated in their respective storage units, they may be driven to a joint accumulation chamber, where all the rejects treated come together prior to being reintroduced into the process. This accumulation unit for treated rejects is connected at its input to the output in the rejects storage and treatment units, and at its output to the water lines of the plant (means for injecting sea, fresh and/or salty water into the photobioreactors).

The photobioreactors may also comprise means for controlling the temperature, which are preferably joint temperature and pH probes very common in the state of the art. They are integrated in line in the network of pipes that carries each photobioreactor, in order to join the different tubes and between one photobioreactors and others, by means of heat pumps of the type condensed by air, by water and/or are geothermal, in order to keep said parameter between 5 °C and 45 °C, as required by the process. Said temperature control or adjustment means are made up of heat pumps of the type condensed by air, condensed by water and/or are geothermal, in order to keep culture in the desired temperature conditions. With the addition of the third concentric outer tube, alongside the two inner tubes between which the culture is found, temperature adjustment needs are reduced considerably, this being one of the aspects that improve the overall performance of the system.

Additionally, the plant may incorporate cleaning and disinfection means, which are arranged in line in the network of pipes and electric pumps responsible for the repositioning and extraction processes for the culture of the photobioreactors, as well as in the rejects recovery processes and exchanges in the different tanks, being selected from the group consisting of ultraviolet rays, active carbon filters and/or ozonation, thus meaning no contaminant element is poured to the environment. also as differentiation, as well as means for collecting accidental leaches and spills, formed by driving means and pumping means up to the storage unit for the reject extracted in all the processes for obtaining biomass, from the units for extracting lipids and/or fatty acids and/or added-value products and the thermochemical treatment units. The cleaning and disinfection means are incorporated into the entire pipe system and remaining system elements. These systems differ from the usual ones used up until now, since they do not emit any toxic substance into the environment. The plant, in order to collect accidental leaches and spills, comprises networks of underground pipes, catch basins and electric pumps, which in the event of the culture accidentally being spilt, will be sent to the corresponding reject deposits in order to be treated and subsequently reused in the culture.

The plant optionally but not exclusively comprises means for controlling the process formed by sensors, flow meters, electrically operated valves and common elements in an SCADA (Supervisory Control and Data Acquisition) system. These elements are located in the networks of pipes and tanks and are responsible for the independent operation of the plant, according to the operating conditions thereof and owing to the electrically operated valves and probes installed.

In the most preferred of all embodiments, the plant described in any of the variations thereof also includes a biofuel production unit and a rejection by means of cavitation, decantation, pressing and/or heat treatment for the biocrude obtained in the thermochemical treatment unit in which said bio-crude is obtained, to which it is connected. This unit may also be connected to a reject storage unit, which works and is connected to the system thereof, in the same conditions as the storage and conditioning unit for the first and second rejects.

Ultimately, the installation described, wherein a wide range of products of commercial interest are generated from biomass, operates as a biorefinery.

Another aspect of the invention refers to food, feed, cosmetic, hygiene, beauty, supplement and alimentary, products, as well as office supplies, paints, plastic arts, DIY, building and cleaning materials and farm products, obtained by the process of the invention.

Examples of food products obtained by the process of the invention are:
1. Water with microalgae, having as a reference Mochique water, with a 0.0001-30% inclusion of microalgae and/or nanoalgae.
2. Biogenic water with microalgae, with a 0.0001-30% inclusion of microalgae and/or nanoalgae.
3. Refreshing beverage with microalgae, having as a reference slush ("granizado"), with a 0.0001-30% inclusion of microalgae and/or nanoalgae.
4. Refreshing beverage with microalgae, having as a reference Aquarius, with a 0.0001-30% inclusion of microalgae and/or nanoalgae.
5. Refreshing beverage with microalgae, having as a reference Compal nectar (min 40% fruit), with a 0.0001-30% inclusion of microalgae and/or nanoalgae.
6. Refreshing beverage with microalga, having as a reference the Bloody mary or tomato juice, with a 0.0001-30% inclusion of microalgae and/or nanoalgae.
7. Microalgae refreshing beverage, with a 0.0001-30% inclusion of microalgae and/or nanoalgae.
8. Vegetable beverages, with a 0.0001-30% inclusion of microalgae and/or nanoalgae.
9. Energy microalgae beverage, having as a reference Isostar, with a 0.0001-30% inclusion of microalgae and/or nanoalgae.
10. Craft beer with microalgae, with a 0.0001-30% inclusion of microalgae and/or nanoalgae.
11.Industrial beer with microalgae, with a 0.0001-30% inclusion of microalgae and/or nanoalgae.
12.Tigernut milk drink with microalgae, having as a reference Horchata de Chufa, with a 0.0001-30% inclusion of microalgae and/or nanoalgae.
13.Juice with microalgae, having as a reference juices as Compal de frutas, with a 0.0001-30% inclusion of microalgae and/or nanoalgae.
14.Milk for transition period with microalgae, having as a reference Aptamil, with a 0.0001-30% inclusion of microalgae and/or nanoalgae.
15.Milk for growth period with microalgae, having as a reference Aptamil junior, with 0.0001-30% inclusion of microalgae and/or nanoalgae.
16.Milk for adaptation period with microalgae, having as a reference Mimosa milk (enriched in calcium), with a 0.0001-30% inclusion of microalgae and/or nanoalgae.
17. Sorbet with microalgae, with a 0.0001-30% inclusion of microalgae and/or nanoalgae.
18. Water ice cream with microalgae, having as a reference Calipo, with a 0.0001-30% inclusion of microalgae and/or nanoalgae.
19.Ice cream with microalgae, having as a reference Carted'Or, with 0.0001-30% inclusion of microalgae and/or nanoalgae.
20. Wine with microalgae, with 0.0001-30% inclusion of microalgae and/or nanoalgae.
21.Soy/almonds/rice milk beverages and others with microalgae, with 0.0001-30% inclusion of microalgae and/or nanoalgae .
22. Flavored milk with microalgae, having as a reference the coffe or chocolate with milk, with a 0.0001-30% inclusion of microalgae and/or nanoalgae.
23.Fermented milk with microalgae, with a 0.0001-30% inclusion of microalgae and/or nanoalgae.
24.Kombucha with microalgae, with a 0.0001-30% inclusion of microalgae and/or nanoalgae.
25. Liqueur with microalgae, with a 0.0001-30% inclusion of microalgae and/or nanoalgae.
26. Cocoa with microalgae, with a 0.0001-30% inclusion of microalgae and/or nanoalgae.
27. Chocolate with microalgae, with a 0.0001-30% inclusion of microalgae and/or nanoalgae.
28. Coffee with microalgae, with a 0.0001-30% inclusion of microalgae and/or nanoalgae.
29.Soluble coffee with microalgae, with a 0.0001-30% inclusion of microalgae and/or nanoalgae.
30.Guarana with microalgae, with a 0.0001-30% inclusion of microalgae and/or nanoalga.
31.Infusion with microalgae, with a 0.0001-30% inclusion of microalgae and/or nanoalgae.
32.Candies with microalgae, with a 0.0001-30% inclusion of microalgae and/or nanoalgae.
33. Lollipop with microalgae, with a 0.0001-30% inclusion of microalgae and/or nanoalgae.
34.Chewing-gum with microalgae, with a 0.0001-30% inclusion of microalgae and/or nanoalgae.
35. Fruit pastilles or gumdrops with microalgae, with a 0.0001-30% inclusion of microalgae and/or nanoalgae.
36.Syrup of microalgae, with a 0.0001-30% inclusion of microalgae and/or nanoalgae.
37.Caramel sauce with microalgae, with a 0.0001-30% inclusion of microalgae and/or nanoalgae.
38.Molasses with microalgae, with a 0.0001-30% inclusion of microalgae and/or nanoalgae.
39.Sweet of fruit with microalgae, with a 0.0001-30% inclusion of microalgae and/or nanoalgae.
40. Compote with microalgae, with a 0.0001-30% inclusion of microalgae and/or nanoalgae.
41.Meringue with microalgae, with a 0.0001-30% inclusion of microalgae and/or nanoalgae.
42.Jelly with microalgae, with a 0.0001-30% inclusion of microalgae and/or nanoalgae.
43.Turron (nougat candy) with microalgae, with a 0.0001-30% inclusion of microalgae and/or nanoalgae.
44.Cakes with microalgae, with a 0.0001-30% inclusion of microalgae and/or nanoalgae.
45. Marzipan and other sweets with microalgae, with a 0.0001-30% inclusion of microalgae and/or nanoalgae.
46.Salt with microalgae, with a 0.0001-30% inclusion of microalgae and/or nanoalgae.
47.Microalgae in powder or in tablets, with a 0.0001-30% inclusion of microalgae and/or nanoalgae.
48.Cubes for soups with microalgae, with a 0.0001-30% inclusion of microalgae and/or nanoalgae.
49.Aromatic herbs with microalgae, with a 0.0001-30% inclusion of microalgae and/or nanoalgae.
50. Soya sauce with microalgae, with a 0.0001-30% inclusion of microalgae and/or nanoalgae.
51.Miso with microalgae, with a 0.0001-30% inclusion of microalgae and/or nanoalgae.
52.Souces with microalgae, with a 0.0001-30% inclusion of microalgae and/or nanoalgae.
53.Olives with microalgae, with a 0.0001-30% inclusion of microalgae and/or nanoalgae.
54. Fruit purees with microalgae, with a 0.0001-30% inclusion of microalgae and/or nanoalgae.
55. Seeds with microalgae, with a 0.0001-30% inclusion of microalgae and/or nanoalgae.
56.Tinned food with microalgae, with a 0.0001-30% inclusion of microalgae and/or nanoalgae.
57.Olive/soya/sunflower/palma/colza/coconut oil and others with microalgae, with a 0.0001-30% inclusion of microalgae and/or nanoalgae.
58.Oil with pigments and/or carotenes from microalgae, with a 0.0001-30% inclusion of microalgae and/or nanoalgae.
59.Oil with fatty acids, with a 0.0001-30% inclusion of microalgae and/or nanoalgae.
60. Margarine with microalgae, with a 0.0001-30% inclusion of microalgae and/or nanoalgae.
61. Peanut butter with microalgae, with a 0.0001-30% inclusion of microalgae and/or nanoalgae.
62.Flours with microalgae, with a 0.0001-30% inclusion of microalgae and/or nanoalgae.
63. Biscuits with microalgae, with a 0.0001-30% inclusion of microalgae and/or nanoalgae.
64. Crackers with microalgae, with a 0.0001-30% inclusion of microalgae and/or nanoalgae.
65. Bread (all cereals) with microalgae, with a 0.0001-30% inclusion of microalgae and/or nanoalgae.
66. Pasta with microalgae, with a 0.0001-30% inclusion of microalgae and/or nanoalgae.
67. Noodles with microalgae, with a 0.0001-30% inclusion of microalgae and/or nanoalgae.
68. Gelatin of microalgae, with a 0.0001-30% inclusion of microalgae and/or nanoalgae.
69.Cheesse with microalgae, with a 0.0001-30% inclusion of microalgae and/or nanoalgae.
70.Yogurt with microalgae, with a 0.0001-30% inclusion of microalgae and/or nanoalgae.
71. Butter with microalgae, with a 0.0001-30% inclusion of microalgae and/or nanoalgae
72. Bars of fish and breaded fishes with microalgae, with a 0.0001-30% inclusion of microalgae and/or nanoalgae.
73. Prepared and derivated from meat products with microalgae, with a 0.0001-30% inclusion of microalgae and/or nanoalgae.
74. Prepared and derivated from fish products with microalgae, with a 0.0001-30% inclusion of microalgae and/or nanoalgae.
75. Prepared and derivated from shellfish meat products with microalgae, with a 0.0001-30% inclusion of microalgae and/or nanoalgae.
76. Nuggets with microalgae, with a 0.0001-30% inclusion of microalgae and/or nanoalgae.
77. Honey with microalgae, with a 0.0001-30% inclusion of microalgae and/or nanoalgae.
78.Milk-shakes with microalgae, with a 0.0001-30% inclusion of microalgae and/or nanoalgae.
79.Pâté with microalgae, with a 0.0001-30% inclusion of microalgae and/or nanoalgae.
80.Mayonnaise with microalgae, with a 0.0001-30% inclusion of microalgae and/or nanoalgae.
81.Humus with microalgae, with a 0.0001-30% inclusion of microalgae and/or nanoalgae.
82.Bechamel with microalgae, with a 0.0001-30% inclusion of microalgae and/or nanoalgae.
83.Tofu with microalgae, with a 0.0001-30% inclusion of microalgae and/or nanoalgae.
84.Mashed potatoes with microalgae, with a 0.0001-30% inclusion of microalgae and/or nanoalgae.
85.Prepared and derivated from vegetables products with microalgae, with a 0.0001-30% inclusion of microalgae and/or nanoalgae.
86.Vegetal sausages with microalgae, with a 0.0001-30% inclusion of microalgae and/or nanoalgae.
87.Seasoning for paella and pastas with microalgae, with a 0.0001-30% inclusion of microalgae and/or nanoalgae.
88.Soups (dehydrated or not) with microalgae, with a 0.0001-30% inclusion of microalgae and/or nanoalgae.
89.Protein supplements with microalgae, with a 0.0001-30% inclusion of microalgae and/or nanoalgae.
90.Nutritional meal substitute with microalgae, with a 0.0001-30% inclusion of microalgae and/or nanoalgae.
91. Energy bars with microalgae, with a 0.0001-30% inclusion of microalgae and/or nanoalgae.
92. Popcorn with microalgae, with a 0.0001-30% inclusion of microalgae and/or nanoalgae
93.Cereals for breakfast with microalgae, with a 0.0001-30% inclusion of microalgae and/or nanoalgae.
94.Cereals with microalgae, with a 0.0001-30% inclusion of microalgae and/or nanoalgae.
95.Chips and derivates with microalgae, with a 0.0001-30% inclusion of microalgae and/or nanoalgae.
96. Extruded products and derivates with microalgae, with a 0.0001-30% inclusion of microalgae and/or nanoalgae.
97.Snacks with microalgae, with a 0.0001-30% inclusion of microalgae and/or nanoalgae.

Examples of feed products obtained by the process of the invention are:
98.Feed with microalgae for race horses, with a 0.0001-30% inclusion of microalgae and/or nanoalgae.
99. Feed with microalgae for sport horses, with a 0.0001-30% inclusion of microalgae and/or nanoalgae.
100. Feed with microalgae for saddle horses, with a 0.0001-30% inclusion of microalgae and/or nanoalgae.
101. Feed with microalgae for draught horses, with a 0.0001-30% inclusion of microalgae and/or nanoalgae.
102. Feed with microalgae for stallions, with a 0.0001-30% inclusion of microalgae and/or nanoalgae.
103. Feed with microalgae for donkeys, with a 0.0001-30% inclusion of microalgae and/or nanoalgae.
104. Feed with microalgae for dairy cow in gestation period, with a 0.0001-30% inclusion of microalgae and/or nanoalgae.
105. Feed with microalgae for dairy cow in lactation period, with a 0.0001-30% inclusion of microalgae and/or nanoalgae.
106. Feed with microalgae for dairy cows in dry period, with a 0.0001-30% inclusion of microalgae and/or nanoalgae.
107. Feed with microalgae for heifers, with a 0.0001-30% inclusion of microalgae and/or nanoalgae.
108. Feed with microalgae for calves, with a 0.0001-30% inclusion of microalgae and/or nanoalgae.
109. Feed with microalgae for beef cattle in gestation period, with a 0.0001-30% inclusion of microalgae and/or nanoalgae.
110. Feed with microalgae for fattening bullocks, with a 0.0001-30% inclusion of microalgae and/or nanoalgae.
111. Feed with microalgae for bulls, with a 0.0001-30% inclusion of microalgae and/or nanoalgae.
112. Feed with microalgae for sows in gestation period, with a 0.0001-30% inclusion of microalgae and/or nanoalgae.
113. Feed with microalgae for breeding sows, with a 0.0001-30% inclusion of microalgae and/or nanoalgae.
114. Feed with microalgae for piglets, with a 0.0001-30% inclusion of microalgae and/or nanoalgae.
115. Feed with microalgae for growing and finishing pigs, with a 0.0001-30% inclusion of microalgae and/or nanoalgae.
116. Feed with microalgae for wild boars, with a 0.0001-30% inclusion of microalgae and/or nanoalgae.
117. Feed with microalgae for boars, with a 0.0001-30% inclusion of microalgae and/or nanoalgae.
118. Feed with microalgae for sheep in gestation period, with a 0.0001-30% inclusion of microalgae and/or nanoalgae.
119. Feed with microalgae for pre-weaning lambs, with a 0.0001-30% inclusion of microalgae and/or nanoalgae.
120. Feed with microalgae for dairy sheep, with a 0.0001-30% inclusion of microalgae and/or nanoalgae.
121. Feed with microalgae for meat sheep and muttons, with a 0.0001-30% inclusion of microalgae and/or nanoalgae.
122. Feed with microalgae for goats in gestation period, with a 0.0001-30% inclusion of microalgae and/or nanoalgae.
123. Feed with microalgae for pre-weaning goats, with a 0.0001-30% inclusion of microalgae and/or nanoalgae.
124. Feed with microalgae for dairy goats, with a 0.0001-30% inclusion of microalgae and/or nanoalgae.
125. Feed with microalgae for meat goats, with a 0.0001-30% inclusion of microalgae and/or nanoalgae.
126. Feed with microalgae for laying hens, with a 0.0001-30% inclusion of microalgae and/or nanoalgae.
127. Feed with microalgae for chicken, with a 0.0001-30% inclusion of microalgae and/or nanoalgae.
128. Feed with microalgae for chick, with a 0.0001-30% inclusion of microalgae and/or nanoalgae.
129. Feed with microalgae for turkeys, with a 0.0001-30% inclusion of microalgae and/or nanoalgae.
130. Feed with microalgae for ducks, with a 0.0001-30% inclusion of microalgae and/or nanoalgae.
131. Feed with microalgae for partridges, with a 0.0001-30% inclusion of microalgae and/or nanoalgae.
132. Feed with microalgae for ostrichs, with a 0.0001-30% inclusion of microalgae and/or nanoalgae.
133. Feed with microalgae for pigeons, with a 0.0001-30% inclusion of microalgae and/or nanoalgae.
134. Feed with microalgae for rabbits, with a 0.0001-30% inclusion of microalgae and/or nanoalgae.
135. Feed with microalgae for dogs, with a 0.0001-30% inclusion of microalgae and/or nanoalgae.
136. Feed with microalgae for cats, with a 0.0001-30% inclusion of microalgae and/or nanoalgae.
137. Feed with microalgae for reptiles, like iguana, bearbed dragon and other reptiles in captivity, with a 0.0001-30% inclusion of microalgae and/or nanoalgae.
138. Feed with microalgae for lagomorphs, like european rabbits and others kept as pets, with a 0.0001-30% inclusion of microalgae and/or nanoalgae.
139. Feed with microalgae for rodents, like hamsters, esquirrel and others kept as pets, with a 0.0001-30% inclusion of microalgae and/or nanoalgae.
140. Feed with microalgae for mustelids, with a 0.0001-30% inclusion of microalgae and/or nanoalgae.
141. Feed with microalgae for psitacidae, lilke parrots, macaws and other psitacidae in captivity.
142. Feed with microalgae for songbirds and ornamentals birds, like canary and zebra finch in captivity, with a 0.0001-30% inclusion of microalgae and/or nanoalgae.
143. Feed with microalgae for ornamental fishes, with a 0.0001-75% inclusion of microalgae and/or nanoalgae.
144. Feed with microalgae for amphibians, with a 0.0001-30% inclusion of microalgae and/or nanoalgae.
145. Feed with microalgae for hedgehogs, with a 0.0001-30% inclusion of microalgae and/or nanoalgae.
146. Feed with microalgae for aquaculture larvaes, with a 0.0001-50% inclusion of microalgae and/or nanoalgae.
147. Feed with microalgae for aquaculture fingerlings, with a 0.0001-50% inclusion of microalgae and/or nanoalgae.
148. Feed with microalgae for rotifers, with a 0.0001-90% inclusion of microalgae and/or nanoalgae.
149. Feed with microalgae for mollusk, with a 0.0001-90% inclusion of microalgae and/or nanoalgae.
150. Feed with microalgae for crustaceans, with a 0.0001-90% inclusion of microalgae and/or nanoalgae.
151. Feed with microalgae for fish farm (fresh and salt water), with a 0.0001-90% inclusion of microalgae and/or nanoalgae.
152. Salt blocks with microalgae, with a 0.0001-30% inclusion of microalgae and/or nanoalgae.
153. Feed with microalgae for camels, with a 0.0001-30% inclusion of microalgae and/or nanoalgae.
154. Feed with microalgae for water birds, like swans, ducks, geese, pelicans, penguins, albatross and other water birds (including seabirds), with a 0.0001-30% inclusion of microalgae and/or nanoalgae.
155. Feed with microalgae for shorebirds like hoopoes and others, with a 0.0001-30% inclusion of microalgae and/or nanoalgae.
156. Feed with microalgae for non domestic birds like raven, rook, blackbirds, swalows and others, with a 0.0001-30% inclusion of microalgae and/or nanoalgae.
157. Feed with microalgae for birds of prey, like eagle, white tailed kite and others, with a 0.0001-30% inclusion of microalgae and/or nanoalgae.
158. Feed with microalgae for birds ciconiforms, like storks, flamingos and others, with a 0.0001-30% inclusion of microalgae and/or nanoalgae.
159. Feed with microalgae for common moorhen, emus and other non domestic birds, with a 0.0001-30% inclusion of microalgae and/or nanoalgae.
160. Feed with microalgae for sea mammals, like walrus, sea lions, seal, dolphin, amazon river dolphin, orcas, whale, manatee and others, with a 0.0001-30% inclusion of microalgae and/or nanoalgae.
161. Feed with microalgae for non domestic felines, like lions, tigers, lynx, pumas and others, with a 0.0001-30% inclusion of microalgae and/or nanoalgae.
162. Feed with microalgae for non domestic canines, like wolves, foxes and others, with a 0.0001-30% inclusion of microalgae and/or nanoalgae.
163. Feed with microalgae for non domestic ruminants, like american bisons, deers, giraffes, Ilamas and others, with a 0.0001-30% inclusion of microalgae and/or nanoalgae.
164. Feed with microalgae for non domestic equines, with a 0.0001-30% inclusion of microalgae and/or nanoalgae.
165. Feed with microalgae for marsupials with microalgae, like kangaroos and others, with a 0.0001-30% inclusion of microalgae and/or nanoalgae.
166. Feed with microalgae for primates, like macaques, lemurs, chimpanzees and others, with a 0.0001-30% inclusion of microalgae and/or nanoalgae.
167. Feed with microalgae for bats, with a 0.0001-30% inclusion of microalgae and/or nanoalgae.
168. Feed with microalgae for duck-billed platypus, with a 0.0001-30% inclusion of microalgae and/or nanoalgae.
169. Feed with microalgae for ursids, like bears, panda bears and others, with a 0.0001-30% inclusion of microalgae and/or nanoalgae.
170. Feed with microalgae for preservation and ornamental fishes, with a 0.0001-90% inclusion of microalgae and/or nanoalgae.

Examples of cosmetic, hygiene and beauty products obtained by the process of the invention are:
171. Conditioner with microalgae, with a 0.0001-30% inclusion of microalgae and/or nanoalgae.
172. Hair mask with microalgae, with a 0.0001-30% inclusion of microalgae and/or nanoalgae.
173. Shampoo with microalgae, with a 0.0001-30% inclusion of microalgae and/or nanoalgae.
174. Haircare oil with microalgae, having as a reference Kérastase ELIXIR ULTIME Versatile Beautifying Oil, with a 0.0001-30% inclusion of microalgae and/or nanoalgae.
175. Hair serum with microalgae, having as a reference L'Oréal Professionnel Serie Expert Liss Unlimited Serum, Osis Magic Anti Frizz Shine Serum, with a 0.0001-30% inclusion of microalgae and/or nanoalgae.
176. Hair cream with microalgae, having as a reference creme for brushing reconstrutor and protector of L'Oreal professionnel, with a 0.0001-30% inclusion of microalgae and/or nanoalgae.
177. Hair balsam with microalgae, having as a reference Bonacure Excellium Beautifying Balm For Silver & White Hair, with a 0.0001-30% inclusion of microalgae and/or nanoalgae.
178. Hair milk with microalgae, having as a reference Bonacure Excellium Taming Milk For Coarse Mature Hair, with a 0.0001-30% inclusion of microalgae and/or nanoalgae.
179. Hair elixir with microalgae, having as a reference Wella Professionals Enrich Hair Ends Elixir, with a 0.0001-30% inclusion of microalgae and/or nanoalgae.
180. Styling gel with microalgae, having as a reference Osis G Force Strong Styling Gel, with a 0.0001-30% inclusion of microalgae and/or nanoalgae.
181. Dry shampoo with microalgae, having as a reference Fudge Dry Shampoo, with a 0.0001-30% inclusion of microalgae and/or nanoalgae.
182. Mousse hairstyle with microalgae, having as a reference Osis Glamination Plumping Shine Mousse, with a 0.0001-30% inclusion of microalgae and/or nanoalgae.
183. Hair styling spray with microalgae, having as a reference Osis Session Label Salt Spray, FIX FABULOUS of Kerastase - Spray de fixação de precisão, with a 0.0001-30% inclusion of microalgae and/or nanoalgae.
184. Hair styling serum with microalgae, having as a reference Osis Magic Anti Frizz Shine Serum, with a 0.0001-30% inclusion of microalgae and/or nanoalgae.
185. Hair sunscreen with microalgae, having as a reference Bonacure Sun Protect Spray Conditioner, with a 0.0001-30% inclusion of microalgae and/or nanoalgae.
186. Styling wax with microalgae, with a 0.0001-30% inclusion of microalgae and/or nanoalgae.
187. Body oil with microalgae, having as a reference Fushi Organic Rosehip Oil, with a 0.0001-95% inclusion of microalgae and/or nanoalgae.
188. Massage oil with microalgae, having as a reference Rituals Xiu Xi Calming Body & Massage Oil, with a 0.0001-95% inclusion of microalgae and/or nanoalgae.
189. Depilation wax with microalgae, having as a reference Veet Oriental Wax, with a 0.0001-95% inclusion of microalgae and/or nanoalgae.
190. Body cream with microalgae, having as a reference Elizabeth Arden Eight Hour Cream Intensive Moisturizing Body Treatment, with a 0.0001-95% inclusion of microalgae and/or nanoalgae.
191. Body milk with microalgae, having as a reference Caudalie Vinotherapie Nourishing Body Lotion, with a 0.0001-95% inclusion of microalgae and/or nanoalgae.
192. Sunscreen with microalgae, having as a reference sun cream Nívea, with a 0.0001-95% inclusion of microalgae and/or nanoalgae.
193. Emulsion with microalgae, having as a reference Biafine, with a 0.0001-95% inclusion of microalgae and/or nanoalgae.
194. Cream for cellulite with microalgae, having as a reference Depuralina, with a 0.0001-95% inclusion of microalgae and/or nanoalgae.
195. Healing ointment having as a reference Halibut, with a 0.0001-95% inclusion of microalgae and/or nanoalgae.
196. Self-tanners with microalgae, having as a reference L'Oréal Paris Sublime Bronze Self-Tanning, with a 0.0001-95% inclusion of microalgae and/or nanoalgae.
197. Tonning gel with microalgae, having as a reference DECLÉOR Aroma Dynamic Circulagel Refreshing Toning Gel for Legs, with a 0.0001-95% inclusion of microalgae and/or nanoalgae.
198. Cellulite lotion with microalgae, having as a reference SUNDÃRI Gotu Kola And Sacred Lotus Cellulite Lotion, with a 0.0001-95% inclusion of microalgae and/or nanoalgae.
199. Anti-cellulite oil with microalgae, having as a reference Garnier Body Ultimate Anti-Cellulite Oil, with a 0.0001-95% inclusion of microalgae and/or nanoalgae.
200. Treatmen cream with microalgae, having as a reference Cloud 9 Skin Solutions Skin Rehab Scar Minimising Cream, with a 0.0001-95% inclusion of microalgae and/or nanoalgae.
201. Treatment serum with microalgae, having as a reference Palmer's Cocoa Butter Formula Scar Serum, with a 0.0001-95% inclusion of microalgae and/or nanoalgae.
202. Hand cream with microalgae, having as a reference Garnier Skin Naturals Intense Care Multi Usage Moisturising Cream, with a 0.0001-95% inclusion of microalgae and/or nanoalgae.
203. Hand gel with microalgae, having as a reference Green & Spring Antibacterial Hand Gel, with a 0.0001-95% inclusion of microalgae and/or nanoalgae.
204. Hand serum with microalgae, having as a reference Aveda Hand Relief™ Night Renewal Serum, with a 0.0001-95% inclusion of microalgae and/or nanoalgae.
205. Hand balm with microalgae, having as a reference KENZOKI Sensual Hands Balm, with a 0.0001-95% inclusion of microalgae and/or nanoalgae.
206. Hand oil with microalgae, having as a reference SUNDÃRI Neem Hand Oil, with a 0.0001-95% inclusion of microalgae and/or nanoalgae.
207. Feet cream with microalgae, having as a reference moisturising feet cream Caudalie, with a 0.0001-95% inclusion of microalgae and/or nanoalgae.
208. Feet balm with microalgae, having as a reference Rituals Lao Tze Cooling Foot Balm, with a 0.0001-95% inclusion of microalgae and/or nanoalgae.
209. Feet lotion with microalgae, having as a reference feet lotion BURT'S BEES - Mint, with a 0.0001-95% inclusion of microalgae and/or nanoalgae.
210. Pâté treatment with microalgae, with a 0.0001-95% inclusion of microalgae and/or nanoalgae.
211. Anti-cellulite cream with microalgae, having as a reference LAMA DE ALGAS of GUAM, with a 0.0001-95% inclusion of microalgae and/or nanoalgae.
212. Shaving foam with microalgae, having as a reference shaving foam Gillette Series Anti-irritation, with a 0.0001-50% inclusion of microalgae and/or nanoalgae.
213. Shaving gel with microalgae, having as a reference Nivea Men Suave, with a 0.0001-50% inclusion of microalgae and/or nanoalgae.
214. Shaving cream with microalgae, having as a reference Denim shaving cream, with a 0.0001-50% inclusion of microalgae and/or nanoalgae.
215. After-shave with microalgae, having as a reference AVÈNE AFTER SHAVE BALM, with a 0.0001-50% inclusion of microalgae and/or nanoalgae.
216. Bb cream with microalgae, having as a reference Skin 79 DIAMOND THE PRESTIGE BEBLESH BALM, with a 0.0001-50% inclusion of microalgae and/or nanoalgae.
217. Cc cream with microalgae, having as a reference Skin 79 COMPLETE CC CREAM CORRECT, with a 0.0001-50% inclusion of microalgae and/or nanoalgae.
218. Moisturising facial gel with microalgae, having as a reference DARPHIN HYDRASKIN LIGHT - MOISTURISING CREAM GEL, with a 0.0001-50% inclusion of microalgae and/or nanoalgae.
219. Skin cream with microalgae, having as a reference Visible Difference Moisturising Duo from Elizabeth Arden, with a 0.0001-50% inclusion of microalgae and/or nanoalgae.
220. Skin serum with microalgae, having as a reference AVÈNE HYDRANCE OPTIMALE HYDRATING SERUM, with a 0.0001-50% inclusion of microalgae and/or nanoalgae.
221. Facial elixir with microalgae, having as a reference CAUDALIE BEAUTY ELIXIR, with a 0.0001-50% inclusion of microalgae and/or nanoalgae.
222. Facial lotion with microalgae, having as a reference MURAD BLEMISH CONTROL SKIN PERFECTING LOTION, with a 0.0001-50% inclusion of microalgae and/or nanoalgae.
223. Cleansing facial milk with microalgae, having as a reference CAUDALIE GENTLE CLEANSING MILK, with a 0.0001-50% inclusion of microalgae and/or nanoalgae.
224. Cleansing facial gel with microalgae, having as a reference LA ROCHE-POSAY EFFACLAR PURIFYING CLEANSING GEL, with a 0.0001-50% inclusion of microalgae and/or nanoalgae.
225. Facial foam with microalgae, having as a reference BAREMINERALS DEEP CLEANSING FOAM, with a 0.0001-50% inclusion of microalgae and/or nanoalgae.
226. Thermal water with microalgae, having as a reference AVÈNE THERMAL WATER, with a 0.0001-50% inclusion of microalgae and/or nanoalgae.
227. Micellar cleansing water with microalgae, having as a reference GARNIER SKIN MICELLAR CLEANSING WATER, with a 0.0001-50% inclusion of microalgae and/or nanoalgae.
228. Skin toner with microalgae, having as a reference AVÈNE GENTLE TONER, with a 0.0001-50% inclusion of microalgae and/or nanoalgae.
229. Facial mask with microalgae, having as a reference ORIGINS DRINK UP INTENSIVE OVERNIGHT MASK, with a 0.0001-50% inclusion of microalgae and/or nanoalgae.
230. Facial oil with microalgae, having as a reference BALANCE ME RADIANCE FACE OIL, with a 0.0001-90% inclusion of microalgae and/or nanoalgae.
231. Facial exfoliator with micralgas, having as a reference ELEMIS GENTLE ROSE EXFOLIATOR, with a 0.0001-50% inclusion of microalgae and/or nanoalgae.
232. Lip balm with microalgae, having as a reference REN VITA MINERAL™ LIP BALM, with a 0.0001-50% inclusion of microalgae and/or nanoalgae.
233. Eyes creme contour with microalgae, having as a reference CLINIQUE ALL ABOUT EYES CREAM RICH, with a 0.0001-50% inclusion of microalgae and/or nanoalgae.
234. Eye contour mask with microalgae, having as a reference ELEMIS ABSOLUTE EYE MASK, with a 0.0001-50% inclusion of microalgae and/or nanoalgae.
235. Eye contour serum with microalgae, having as a reference ESTÉE LAUDER ADVANCED NIGHT REPAIR EYE SERUM SYNCHRONIZED COMPLEX II, with a 0.0001-50% inclusion of microalgae and/or nanoalgae.
236. Eye contour gel with microalgae, having as a reference ARPHIN ANTI-FATIGUE SMOOTHING EYE GEL, with a 0.0001-50% inclusion of microalgae and/or nanoalgae.
237. Cleansisng soap bar with microalgae, having as a reference AVÈNE COLD CREAM ULTRA RICH SOAP-FREE CLEANSING BAR, with a 0.0001-50% inclusion of microalgae and/or nanoalgae.
238. Shower gel with microalgae, having as a reference CAUDALIE PECHE DE VIGNE SHOWER GEL, with a 0.0001-50% inclusion of microalgae and/or nanoalgae.
239. Bath salts with microalgae, having as a reference The Bodyshop JAPAN YUZU & GREEN TEA BATH SALTS, with a 0.0001-50% inclusion of microalgae and/or nanoalgae.
240. Shower oil with microalgae, having as a reference AROMATHERAPY ASSOCIATES RELAX DEEP RELAX BATH & SHOWER OIL, with a 0.0001-50% inclusion of microalgae and/or nanoalgae.
241. Intimate hygiene gel with microalgae, having as a reference Uriage GYN-PHY, with a 0.0001-50% inclusion of microalgae and/or nanoalgae.
242. Toothpaste with mircroalgas, having as a reference Colgate Total, with a 0.0001-50% inclusion of microalgae and/or nanoalgae.
243. Deodorant with microalgae, having as a reference Nívea ROLL-ON TALC SENSATION, with a 0.0001-50% inclusion of microalgae and/or nanoalgae.
244. Nail polish with microalgae, having as a reference OPI Nail Lacquer, with a 0.0001-50% inclusion of microalgae and/or nanoalgae.
245. Nail polish remover with microalgae, having as a reference Mavala Mild Nail Polish Remover, with a 0.0001-50% inclusion of microalgae and/or nanoalgae.
246. Cuticle remover with microalgae, having as a reference Aveda Cuticle Control, with a 0.0001-50% inclusion of microalgae and/or nanoalgae.
247. Blush with microalgae, having as a reference Lancôme Blush Subtil 02 Nectar, with a 0.0001-50% inclusion of microalgae and/or nanoalgae.
248. Foundation with microalgae, having as a reference Yves Saint Laurent Forever Foundation, with a 0.0001-50% inclusion of microalgae and/or nanoalgae.
249. Concealer with microalgae, having as a reference L'Oréal Paris True Match Skin Tone Matching Correcting Concealer, with a 0.0001-50% inclusion of microalgae and/or nanoalgae.
250. Powder with microalgae, having as a reference Burberry Skin Nude Powder, with a 0.0001-50% inclusion of microalgae and/or nanoalgae.
251. Eyeliner with microalgae, having as a reference Rimmel Exaggerate Liquid Eyeliner, with a 0.0001-50% inclusion of microalgae and/or nanoalgae.
252. Eyeshadow primer with microalgae, having as a reference NYX Proof It! Waterproof Eyeshadow Primer, with a 0.0001-50% inclusion of microalgae and/or nanoalgae.
253. Eyeshadow with microalgae, having as a reference Estée Lauder Pure Color Envy Sculpting EyeShadow 5-Color Palette, with a 0.0001-50% inclusion of microalgae and/or nanoalgae.
254. Make-up primer with microalgae, having as a reference Lancôme La Base Pro Hydra Glow Illuminating Make-Up Primer, with a 0.0001-50% inclusion of microalgae and/or nanoalgae.
255. Illuminator with microalgae, having as a reference NARS Illuminator, with a 0.0001-50% inclusion of microalgae and/or nanoalgae.
256. Lipstick with microalgae, having as a reference Rimmel The Only 1 Lipstick, with a 0.0001-50% inclusion of microalgae and/or nanoalgae.
257. Lipgloss with microalgae, having as a reference Pür Minerals Château Kisses Plumping Lip Gloss, with a 0.0001-50% inclusion of microalgae and/or nanoalgae.
258. Lip primer with microalgae, having as a reference Too Faced Lip Insurance Lip Primer, with a 0.0001-50% inclusion of microalgae and/or nanoalgae.
259. Facial masck with microalgae, having as a reference Rimmel Wonder'full Waterproof Mascara, with a 0.0001-50% inclusion of microalgae and/or nanoalgae.
260. Tinted moisturizer with microalgae, having as a reference Clinique Moisture Surge Tinted Moisturizer, with a 0.0001-50% inclusion of microalgae and/or nanoalgae.
261. Body glue with microalgae, having as a reference Pink Body Glue by Ybody, with a 0.0001-50% inclusion of microalgae and/or nanoalgae.
262. Body paint with microalgae, having as a reference Kryolan AQUACOLOR LIQUID, with a 0.0001-50% inclusion of microalgae and/or nanoalgae.
263. Makeup remover with microalgae, having as a reference Shiseido The Skincare Essentials Instant Eye and Lip Makeup Remover, with a 0.0001-50% inclusion of microalgae and/or nanoalgae.

Examples of supplements and alimentary compounds obtained by the process of the invention are:
264. Vitamins from microalgae, 100% of microalgae and/or nanoalgae.
265. Proteins from microalgae, 100% of microalgae and/or nanoalgae.
266. Antioxidants from microalgae, 100% of microalgae and/or nanoalgae.
267. Omega -3 from microalgae, 100% of microalgae and/or nanoalgae.
268. PUFAs from microalgae, 100% of microalgae and/or nanoalgae.
269. Minerals from microalgae, 100% of microalgae and/or nanoalgae.
270. DHA from microalgae, 100% of microalgae and/or nanoalgae.
271. EPA from microalgae, 100% of microalgae and/or nanoalgae.
272. Beta-carotenes from microalgae, 100% of microalgae and/or nanoalgae.
273. Vitamins and minerals from microalgae, 100% of microalgae and/or nanoalgae.
274. Chlorophyll from microalgae, 100% of microalgae and/or nanoalgae.
275. Amino-acids from microalgae, 100% of microalgae and/or nanoalgae.
276. Peptide and polypeptides, 100% of microalgae and/or nanoalgae.
277. Polysaccharides from microalgae, 100% of microalgae and/or nanoalgae.

Examples of office supplies, paints and plastic arts obtained by the process of the invention are:
278. Pigments of microalgae, 100% of microalgae and/or nanoalgae.
279. Acrylic paint with microalgae, having as a reference College Acrylic, with a 0.0001-50% inclusion of microalgae and/or nanoalgae or its constituents.
280. Oil paints with microalgae, having as a reference Akaofmie Oil, with a 0.0001-50% inclusion of microalgae and/or nanoalgae or its constituents.
281. Watercolor with microalgae, having as a reference Aguarela Van Gogh, with a 0.0001-50% inclusion of microalgae and/or nanoalgae or its constituents.
282. Chalk with microalgae, with a 0.0001-50% inclusion of microalgae and/or nanoalgae or its constituents.
283. Oleo pastel with microalgae, with a 0.0001-50% inclusion of microalgae and/or nanoalgae or its constituents.
284. Gouache paint with microalgae, having as a reference Caran D' Ache Guache, with a 0.0001-50% inclusion of microalgae and/or nanoalgae or its constituents.
285. Varnish vitral with microalgae, having as a reference VERNIZ VITRAL AMARELO OURO ACRILEX, with a 0.0001-50% inclusion of microalgae and/or nanoalgae or its constituents.
286. Paint for clothes with microalgae, having as a reference Tinta Tecido Acrilex, with a 0.0001-50% inclusion of microalgae and/or nanoalgae or its constituents.
287. Varnish with microalgae, having as a reference CIN SINTECIN BRILHANTE, with a 0.0001-50% inclusion of microalgae and/or nanoalgae or its constituents.
288. Pencil colour with microalgae, having as a reference Lápis Cor Maped, with a 0.0001-50% inclusion of microalgae and/or nanoalgae or its constituents.
289. Watercolour pencil with microalgae, having as a reference Pencil ALBRECHT DÜRER of aquarela of Faber-Castell, with a 0.0001-50% inclusion of microalgae and/or nanoalgae or its constituents.
290. Graphite pencil with microalgae, with a 0.0001-50% inclusion of microalgae and/or nanoalgae or its constituents.
291. Ballpoint pen with microalgae, having as a reference Esferográfica Cerrutti, with a 0.0001-50% inclusion of microalgae and/or nanoalgae or its constituents.
292. Crayon with microalgae, having as a reference Lápis Cera Giotto, with a 0.0001-50% inclusion of microalgae and/or nanoalgae or its constituents.
293. Highlighter with microalgae, having as a reference BiC MARC.PERMANENTE, with a 0.0001-50% inclusion of microalgae and/or nanoalgae or its constituents.
294. Charcoal with microalgae, with a 0.0001-50% inclusion of microalgae and/or nanoalgae or its constituents.
295. Ink for inkwell with microalgae, having as a reference Pelikan, with a 0.0001-50% inclusion of microalgae and/or nanoalgae or its constituents.
296. Permanet pen refills with microalgae, with a 0.0001-50% inclusion of microalgae and/or nanoalgae or its constituents.
297. Chinese ink with microalgae, with a 0.0001-50% inclusion of microalgae and/or nanoalgae or its constituents.
298. Sanguinia pencil with microalgae, with a 0.0001-50% inclusion of microalgae and/or nanoalgae or its constituents.
299. Felt pen with microalgae, having as a reference Caran D'Ache Canetas of Feltro, with a 0.0001-50% inclusion of microalgae and/or nanoalgae or its constituents.
300. Glue wtih microalgae, having as a reference Cola UHU Maofira, with a 0.0001-50% inclusion of microalgae and/or nanoalgae or its constituents.

Examples of DIY, building and cleaning materials obtained by the process of the invention are:
301. Paint for walls (indoors and outdoors) of microalgae, having as a reference CIN Tinta aquosa lisa e mate, with a 0.0001-50% inclusion of microalgae and/or nanoalgae or its constituents.
302. Velatura of microalgae, having as a reference Libéron VELATURA ACRÍLlCA TONS COLORIDOS, with a 0.0001-50% inclusion of microalgae and/or nanoalgae or its constituents.
303. Varnish of microalgae, having as a reference Libéron VERNIZ BISTROT, with a 0.0001-50% inclusion of microalgae and/or nanoalgae or its constituents.
304. Softener for cloth with microalgae, having as a reference Amaciador Comfort Essencia, with a 0.0001-50% inclusion of microalgae and/or nanoalgae or its constituents.
305. Air freshener with microalgae, having as a reference Ambientador Brise Refresh, with a 0.0001-50% inclusion of microalgae and/or nanoalgae or its constituents.
306. Cloth detergent with microalgae, having as a reference Detergente Roupa Skip, with a 0.0001-50% inclusion of microalgae and/or nanoalgae or its constituents.
307. Dish detergent with microalgae, having as a reference Detergente Loiça Fairy tudo rm 1, with a 0.0001-50% inclusion of microalgae and/or nanoalgae or its constituents.
308. Odor absorbent with microalgae, having as a reference Absorvente Odor Mister Magic P/frigorifico, with a 0.0001-50% inclusion of microalgae and/or nanoalgae or its constituents.
309. Dish polish with microalgae, having as a reference Abrilhantador Finish Power & Pure, with a 0.0001-50% inclusion of microalgae and/or nanoalgae or its constituents.
310. Salt for dishwasher with microalgae, having as a reference Sal Maquina Loica Calgonit, with a 0.0001-50% inclusion of microalgae and/or nanoalgae or its constituents.
311. Shoe cream with microalgae, having as a reference Creme Calcado Splendor, with a 0.0001-50% inclusion of microalgae and/or nanoalgae or its constituents.
312. Clean leather with microalgae, having as a reference Limpa Couro, BELLA PELLE, with a 0.0001-50% inclusion of microalgae and/or nanoalgae or its constituents.
313. Toilet freshener with microalgae, having as a reference Bloco Auto Matic Pato, with a 0.0001-50% inclusion of microalgae and/or nanoalgae or its constituents.
314. Cream for cleaning with microalgae, having as a reference Creme Cif Ativo, with a 0.0001-50% inclusion of microalgae and/or nanoalgae or its constituents.
315. Gel for cleaning with microalgae, having as a reference Detergente Wc Harpic, with a 0.0001-50% inclusion of microalgae and/or nanoalgae or its constituents.
316. Multiuse cleaning with microalgae, having as a reference Lava Tudo Ajax Fabuloso, with a 0.0001-50% inclusion of microalgae and/or nanoalgae or its constituents.
317. Window cleaning with microalgae, having as a reference Limpa Vidros Ajax, with a 0.0001-50% inclusion of microalgae and/or nanoalgae or its constituents.
318. Oven and furnaces cleaning cream with microalgae, having as a reference Limpador Scotch Brite Vitroceramica, with a 0.0001-50% inclusion of microalgae and/or nanoalgae or its constituents.
319. Degreasers with microalgae, having as a reference Desengordurante Cif Spray, with a 0.0001-50% inclusion of microalgae and/or nanoalgae or its constituents.
320. Desinfectant with microalgae, having as a reference Desinfectante Sanytol Cozinhas, with a 0.0001-50% inclusion of microalgae and/or nanoalgae or its constituents.
321. Metal-cleaning with microalgae, having as a reference Limpa Metais Algodao, duraglit, with a 0.0001-50% inclusion of microalgae and/or nanoalgae or its constituents.
322. Wax with microalgae, having as a reference Cera Novycera, with a 0.0001-50% inclusion of microalgae and/or nanoalgae or its constituents.
323. Detergent for parquet with microalgae, having as a reference Limpa Chao Novycera Madeira Eco, with a 0.0001-50% inclusion of microalgae and/or nanoalgae or its constituents.
324. Wood repair, having as a reference Renovador Madeira/flutuan Alex, with a 0.0001-50% inclusion of microalgae and/or nanoalgae or its constituents.
325. Bleach with microalgae, having as a reference Lixivia Neoblanc, with a 0.0001-50% inclusion of microalgae and/or nanoalgae or its constituents.
326. Knots remover with microalgae, having as a reference Tira Nodoas Blancotex, with a 0.0001-50% inclusion of microalgae and/or nanoalgae or its constituents.

Examples of farm products obtained by the process of the invention are:
327. Seeds with microalgae, with a 0.0001-50% inclusion of microalgae and/or nanoalgae.
328. Biofertilizers with microalgae, with a 0.0001-99% inclusion of microalgae and/or nanoalgae.
329. Phytohormones and growth inductors (phytobiotic) from microalgae, 100% de microalgae and/or nanoalgae.
330. Betaine from microalgae, 100% of microalgae and/or nanoalgae.

### PREFERRED EMBODIMENT OF THE INVENTION

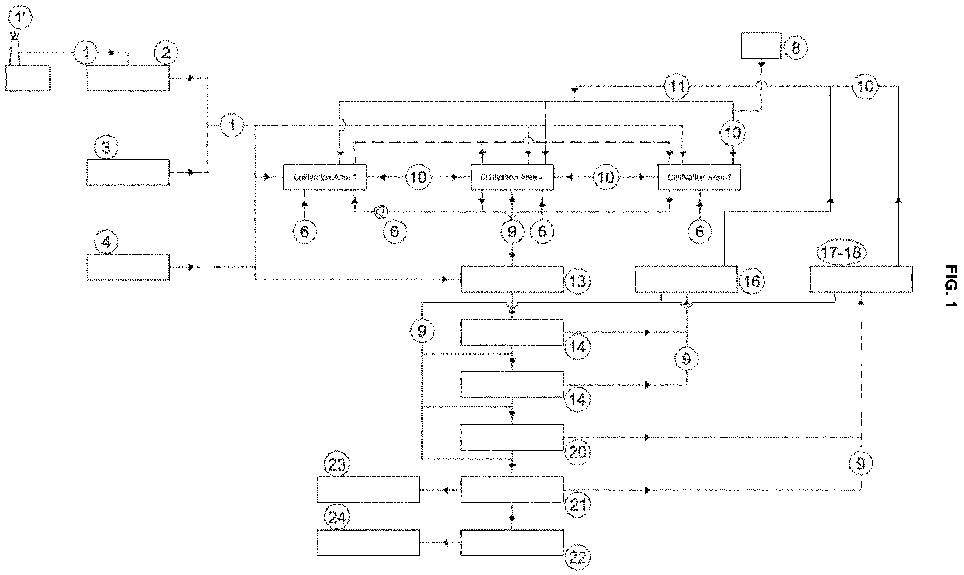

In a very preferred case, as described above and as shown in Figure 1, the biomass production plant comprises:
(1) a unit for collecting, storage and injection of greenhouse gasses coming from smoke stacks of industrial facilities that emit them (1'). This system is formed in a non-limiting manner by take-offs from smoke stacks, duct networks, automatic operating systems, accessories and extractors.
(2) a unit for washing and compressing greenhouse gases, the temperature of which is adjusted if the same is above 40 °C.
(3) a unit for mixing and injecting the greenhouse gases with air, as well as with gases returned from the thermochemical processes and/or surplus of those introduced into the photobioreactors that are consumed in the plant.
(4) A unit for accumulating greenhouse gases, in such a way that if the main supply source thereof fails, the process may carry on. The capacity of these tanks is such that they enable the plant to operate normally, when usual supplies are not provided from between one to four months.
(5) An electric pump in each group of between 2 and 5000 photobioreactors, which makes the fluid in the photobioreactor spin in the opposite direction to that in which the bubbles, with the indicated gasses injected, are introduced;
(6) A set of between 2 and 5000 annular vertical photobioreactors made from plastic materials. In all cases, the photobioreactor has an inner span of less than 5 cm and the ratio achieved by the same in terms of its design and volume (m³)/ surface area (m²) ratio. This ratio may reach up to 180 litres/m². Each photobioreactor is made up of two tubes, one inner tube and one outer tube, between which the algae are cultivated. Outside the two tubes between which the culture is found, a translucent tube is placed, which prevents the direct irradiation of the outer tube containing the culture. Moreover, the section between the inner tube and outer tube containing the culture is divided into X number of longitudinal sections
(7) An automatic tubes cleaning device containing the culture, as described in the previous section.
(8) A unit for collecting, storage and injection and subsequent filtration of sea, fresh or salty water by means of piping systems, electric pumps and filters, as well as control elements. It also has an additional supply water accumulation tank, with a capacity of between one sixth and two sixths of the total capacity of the plant culture volume.
(9) A system of pipes that interconnect the cultivation areas to the culture accumulation and treatment areas and finished product accumulation and treatment areas.
(10) A unit for injecting nutrients into the water current that supplies the photobioreactors, in order to promote the rapid development of unicellular algae. There is a dosage cap for macro and micronutrients and trace metals. Owing to the nutrient probes installed in line in the piping of the process, the corresponding orders will be given to the nutrients pumps, so that they inject the culture as required.
(11) Means for cleaning and disinfecting the plant by means of ultraviolet rays and active carbon filters, in addition to ozonation. They are introduced into all the piping systems and remaining elements in the plant.
(12) Means for collecting upper foam in each one of the photobioreactors. This foam is collected and sent by means of the corresponding system of pipes and electric pumps to the corresponding accumulation tanks, thereby being reintroduced into the process.
(13) Unit for accumulating the culture extracted from the photobioreactors. The capacity thereof is between half and one tenth that of the total volume of the plant. It has stirrers and a supply system with CO₂ and/or compressed air and measurement and control systems, to be integrated into the plant operation, thus making it possible to attain different reactions therein, according to the characteristics of the culture collected.
(14) Mechanical and chemical separation units for separating the unicellular algae culture extracted and accumulated in the unit (13).
(15) Means for controlling the temperature, made up of heat pumps of the type that are condensed by air, in order to keep the culture in temperature conditions of between 5 °C and 45 °C, as required.
(16) Chamber for accumulating rejects from the mechanical and chemical separation process. Between 50 % and 99 % of the culture volume filtered is returned to these deposits for treatment and subsequent reincorporation into the cultivation process. Its capacity is between half and one tenth that of total volume of the plant. They have stirrers and aeration systems and/or CO₂+air input, and measurement and control systems to be integrated into the plant operation.
(17) Chamber for accumulating the rejects generated in the units for extracting lipids and/or fatty acids and/or value-added products and for obtaining bio-crude. All those elements considered to result from the various processes, which do not have an end use, are sent to this tank and integrated back into the system after having undergone corresponding treatment, pH and salinity adjustment, microfiltration, disinfection, nutrient addition and agitation. Their capacity is between half and one tenth that of the plant's total volume. They have stirrers and aeration systems and/or CO₂+air input, and measurement and control systems to be integrated into the plant operation.
(18) Means for collecting accidental spills and leaches in the plant. In the event of this embodiment, the unit in which the spills and leaches are accumulated is the same as the reject accumulation unit. It has a system of underground pipe networks, catch basins and electric pumps. In the event of the culture accidentally being spilt, it is sent to the corresponding unit. This is a tank for rejects to be treated and subsequently reused in the culture. In the event of the present embodiment, said means for collecting and storing spills are the same as those for accumulating rejects generated in the units for extracting lipids and/or fatty acids and/or added-value products.
(19) Means of driving the water currents, culture and products obtained in the process. Said means are electric pumps, which perform all movements of the culture and subsequent products between the different zones described in the above elements.
(20) Unit for extracting lipids and/or fatty acids and/or added-value products.
(21) Thermochemical treatment unit for producing biocrude.
(22) Unit for producing biofuel by means of treating biocrude.
(23) Biocrude and biofuel accumulation tanks.
(24) General plant control means, encompassing all the sensors, flow meters, electrically operated valves and other conventional elements in a SCADA type system, which make it possible to vary the operating conditions of the plant within the different values established for each one of the parameters.

### DESCRIPTION OF THE DRAWINGS

Figure 1.Illustrative diagram of the biomass production plant in accordance with the present invention, according to the preferred embodiment described in the previous section.

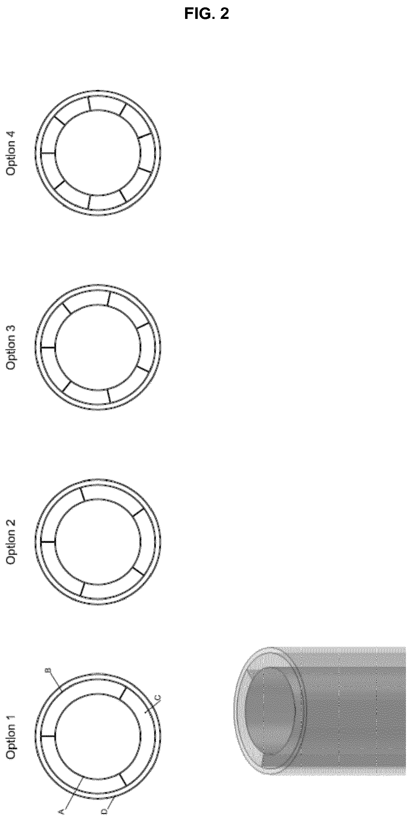

Figure 2.Plan view (and a view in 3D) of alternative configurations of tube photobioreactors to be used in the production plant depending on the number of longitudinal sections into which they are divided: Option 1 with 3 sections, Option 2 with 5 sections; Option 3 with 7 sections and Option 4 with 9 sections. A= Inner tube; B= Outer tube; C= Culture; D= Ventilation tube.

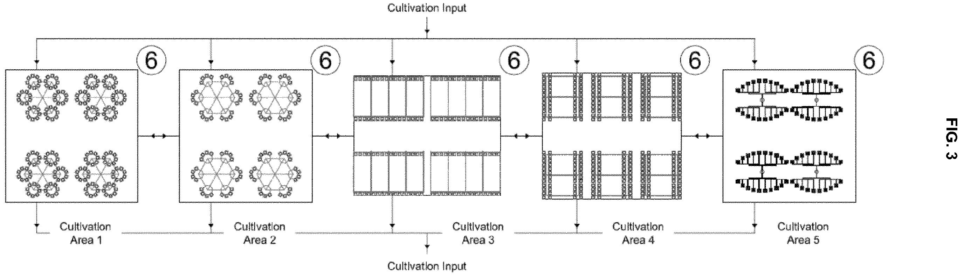

Figure 3.Alternative set of photobioreactors. Different arrangements of the tubes containing the culture, at the time a photobioreactor is formed, are shown in this figure, in such a way that the distances between the same vary, and, depending on their orientation and the placement of a photobioreactor relative to contiguous photobioreactors, first several irradiation values are reached, thereby obtaining an optimised plant suited to all conditions throughout the year depending on its location.

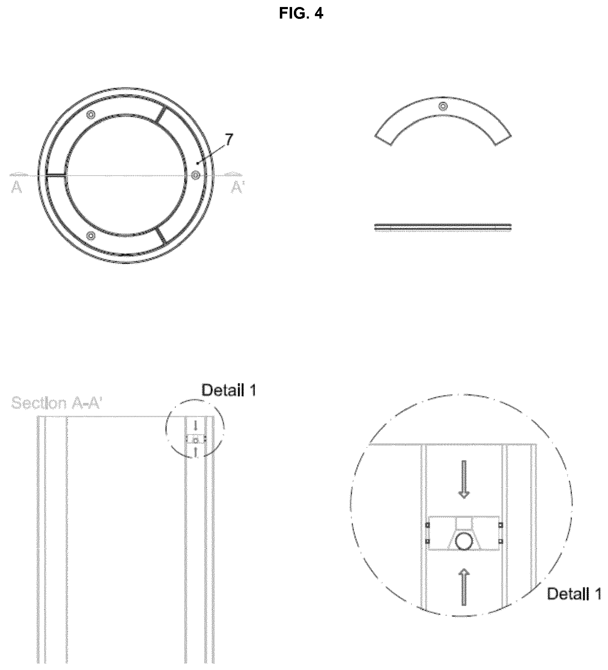

Figure 4.Plan, elevation and transverse views of the cleaning device incorporated inside the photobioreactors tubes.

### EXAMPLES

Three examples of the present invention are described below, which in no case can be considered limiting examples of the invention.

### Example 1:

In Figure 1, the plant designed and the entire process object of the present invention are described. Starting with an estimated cultivation volume of 100 m³, the process is initiated by introducing these 100 m³ of seawater through the mandatory seawater collection system (8). After having disinfected and filtered (11) the same, it is introduced into the cultivation photobioreactor (6), all of the above being carried out at a grade of filtration of less than 1 micron.

The initial strains for inoculating the biomass are also filtered and disinfected (11) prior to being introduced into the photobioreactors (6).

The cultivation area operating system is carried out progressively, in such a way that the photobioreactors (6) are firstly filled with less water thickness sheet, and therefore more productivity, and it is subsequently spread out as the optimal concentration is achieved (between 100 M of cells/ml and 600 M of cells/ml) until the entire cultivation area is obtained, at which point the rest of the process may be started. This step may take from several days to several weeks depending on environmental conditions and the volume and concentration of the initial strain used to begin with.

During this process, and continuously during normal exploitation of the plant, CO₂ is introduced from the gas collection system (1), through its injection means, to the photobioreactors (6), after having been washed in the corresponding unit (2). The amount of gas and compressed air mixture to be introduced depends on the environmental conditions and the state of the culture, however it will be found between the values of 0.2 m<3>of air+CO₂ per cubic meter of culture and 2 m³ of air+CO₂ per cubic meter of culture. As mentioned above, the introduction of air into the system does not necessarily have to be continuous. In an alternative scenario, the culture recirculation pump (5) will act in parallel to the introduction of CO₂ and/or CO₂+air or alone, if said supply were not necessary, given the state of the culture at certain points in time. The overall ratio of CO₂ removed is 1.8 kg/CO₂ per each kg of biomass subsequently produced. Auxiliary CO₂ tanks (4) are anticipated in the state of the art, which may make up for any lack of CO₂ through the usual collection system (1).

Meanwhile, nutrients (10) are introduced into the culture, which are mainly nitrogenous salts (nitrate and ammonium) and phosphates, in the proportion required according to the needs and level of development of the same. The total proportion of nutrients added is between 10 % and 30 % of the biomass obtained. The photobioreactors system has been equipped with an upper foam collection system, in such a way that the nutrients and biomass present in this area are exploited (12).

A culture temperature adjustment system (15) has been installed, although, with the abovementioned improvements made to the photobioreactors, in terms of incorporating a third outer tube, the use thereof will not be necessary during 98 % of the plant operation period. The working temperature ranges greatly, preferably being at between 15 °C and 35 °C. Moreover, an accidental spill collection system (18) has been installed, which spill would be introduced back into the system after appropriate treatment (11) thereof.

Once the optimal degree of culture concentration has been achieved, a daily extraction comprised between 2 % and 50 %, preferably 25 % of the total culture, begins, i.e., between 2 and 50 m³ (9). Meanwhile, despite the fact that most of the water that returns to the cultivation areas, 2 % of that which is extracted, is supplied as new water through the seawater collecting system (8).

The culture, at optimal concentrations, is taken to the accumulation tanks (13). This step feeds the decantation/filtration and/or centrifugation equipment (14) which return part of the culture, at between 50 % and 99 % of the total, to the rejection tanks (16), in order for them to be subsequently treated and introduced back into the culture (6). After this stage, a biomass is obtained, which has an approximate weight of between 40 kg/day and 110 kg/day and a humidity of between 50 % and 90 %, suitable for the subsequent introduction thereof into the next process, without any drying process being required.

The next step of the process is extracting lipids and/or fatty acids and/or added-value products (20), in which between 4 kg/day and 10 kg/day of added-value products are produced.

This is followed by the thermochemical transformation process (21), in which between 20 % and 60 % of bio-crude, between 10 % and 30 % of water, between 1 % and 10 % of char and between 1 % and 30 % of various gases are obtained. This step can be performed with or without a catalyst, and the conditions range between 250 °C and 350 °C and between 150 bar and 210 bar. The processing time can vary between 1 minute and 120 minutes. After this process, a portion of the resulting products are sent back to tanks to be treated and subsequently introduced into the process, and the portion of product obtained as bio-crude goes on to the final conversion to biofuel treatment (22). Different tanks (23) accumulate the products obtained in one or another process prior to their final destination. Finally, between 20 and 50 kg/day of bio-crude are obtained, with a CO₂ conversion of between 72 and 198 kg/day.

## Claims

1. Biomass and products derived thereof obtained by a process for producing biomass and products derived thereof by means of cultivating unicellular algae in aqueous medium fed with a CO₂ current in a set of photobioreactors, said process comprising:
- injecting a current of sea, salty or fresh water into the photobioreactors as a culture medium for the algae, previously enriched with nutrients, microfiltered and disinfected,
- injecting a strain of at least one species of unicellular algae into the photobioreactors, and putting said strain in contact with the enriched water current under light conditions for the culture to grow by means of photosynthesis;
- injecting a current of gases containing CO₂ into the photobioreactors,
**characterised in that** it further comprises:
- extracting part of the algae culture from the photobioreactors, storing in an accumulation tank and sending to a separate unit to extract the biomass by means of mechanical and/or chemical separation, such that the obtained biomass has a humidity degree comprised between 50 % and 90 %;
- subjecting the wet biomass to decantation and/or cavitation in a separate unit in order to extract part of the lipids and/or fatty acids and other added-value products along with a first reject, passing the remaining biomass on to the following step;
- subjecting the remaining biomass from the preceding step to direct, low-temperature, thermal liquefaction in a separate unit in order to produce a biocrude and a second reject, all this at a temperature comprised between 250 °C and 350 °C and a pressure comprised between 150 and 210 bar, both limits included, for a time comprised between 1 and 120 minutes;
- recovering the rejects from both the step of lipid, fatty acid and other added-value product extraction and the subsequent step of thermochemical treatment under subcritical conditions, storing each reject in separate chambers and subjecting to physicochemical treatment based on adjusting salinity, pH, microfiltration, disinfection and addition of nutrients, for subsequent reintroduction into the culture.

2. Biomass and products derived thereof according to claim 1, wherein the gas current in the process is a CO₂ current or a mixture of gases from a combustion process.

3. Biomass and products derived thereof according to any of claims 1 or 2, wherein the gas current in the process is cooled, washed, compressed and the CO₂ is separated from the other gases prior to introduction thereof into the photobioreactors.

4. Biomass and products derived thereof according to any of claims 1 to 3, wherein the gas current in the process is injected in a radial and discontinuous manner into the photobioreactor, in pulses lasting between 1 and 3600 seconds at time intervals comprised between 1 and 3600 seconds, both limits included.

5. Biomass and products derived thereof according to any of claims 1 to 4, wherein the gas current in the process is injected in an amount comprised between 0,2 m³/m³ of culture and 2 m³/m³ of culture.

6. Biomass and products derived thereof according to any of claims 1 to 5, wherein the nutrients with which the water current is enriched in the process are macronutrients, micronutrients and/or trace elements, in a percentage comprised between 10 % and 30 % by weight of obtained biomass.

7. Biomass and products derived thereof according to any of claims 1 to 6, wherein the nutrients in the process are selected from nitrogenous salts such as nitrate and ammonium, phosphates and any combination thereof.

8. Biomass and products derived thereof according to any of claims 1 to 7, wherein the culture in the process is kept inside the photobioreactors at a temperature comprised between 5 °C and 45 °C.

9. Biomass and products derived thereof according to any of claims 1 to 8, wherein the algae culture in the process is kept inside the photobioreactors until a concentration comprised between 100 million cells/ml and 600 million cells/ml is reached.

10. Biomass and products derived thereof according to any of claims 1 to 9, wherein the mechanical separation for obtaining the biomass is carried out in the process by means of one of the processes selected from the group consisting of centrifugation, super-centrifugation, decantation and/or filtration, and the chemical separation is carried out by means of flocculation.

11. Biomass and products derived thereof according to any of claims 1 to 10, wherein the algae strain in the process is selected from the group consisting of *Cyanobacteria, Chlorophyceae, Rhodophyceae, Eustigmatophyceae,* diatoms, *Dinophyceae, Bacillariophyceae, Xanthophytes* and *Phaeophyceae,* and any combination thereof.

12. Biomass and products derived thereof according to any of claims 1 to 11, wherein the biocrude is subjected in the process to cavitation, decantation, pressing and/or thermal treatment in a separate unit in order to obtain biofuel and a reject.

13. Biomass and products derived thereof according to any of claims 1 to 12, wherein the products derived thereof are selected from the list consisting of: lipids, fatty acids, biocrude, biofuel, vitamins, antioxidants or hormones.

14. Non-therapeutical use of the biomass and products derived thereof according to any of claims 1 to 13 in the agro-food, cosmetic or pharmaceutical industry.

15. Biomass and products derived thereof according to any of claims 1 to 13 for use in medicine.

16. Use of the biomass and products derived thereof according to any of claims 1 to 13 for the manufacture of foods for human or animal consumption, beverages, food supplements, nutritional compositions, cosmetics, hygiene products, paints, dyes, pens, pencils, felt-tip pens, cleaning products, do-it-yourself products, construction materials or agricultural products.
